# FASCICULE DE BREVET EUROPEEN

(11) **EP 2 879 663 B1**
(45) Date de publication et mention de la délivrance du brevet: **19.07.2023**
(21) Numéro de dépôt: 13745040.9
(22) Date de dépôt: 30.07.2013
(51) Int. Cl.: A61K 9/00, A61K 9/24, A61K 31/465, A61P 25/34

(54) **PASTILLES A CINETIQUES MULTIPLES DE LIBERATION DE PRINCIPES ACTIFS**
LUTSCHTABLETTEN MIT MEHRFACHER FREISETZUNGSKINETIK FÜR WIRKSTOFFE
LOZENGES WITH MULTIPLE RELEASE KINETICS FOR ACTIVE INGREDIENTS

(30) Priorité: 30.07.2012 FR 1257389
(43) Date de publication de la demande: 10.06.2015
(73) Titulaire: PIERRE FABRE MEDICAMENT, 81500 Lavaur (FR)
(72) Inventeur: BERTHOUMIEU, Didier, F-31290 Villenouvelle (FR); LAUR, Clément, F-81110 Lescout (FR); CORDOLIANI, Jean-François, F-31570 Sainte Foy d'Aigrefeuille (FR)
(74) Mandataire: Regimbeau
(86) Numéro de dépôt international: PCT/EP2013/066032
(87) Numéro de publication internationale: WO 2014/020039

(56) Documents cités:
- EP-A1- 2 233 134
- WO-A1-01/37814
- WO-A1-03/039518
- GB-A- 2 230 439
- US-A- 5 783 207
- US-A1- 2002 002 189
- US-A1- 2004 101 543
- TIRPUDE RAKESH N ET AL: "Drug Multiparticulate Production and Coating Technology - A Review", RESEARCH JOURNAL OF PHARMACY AND TECHNOLOGY, A & V PUBLICATIONS, INDIA, vol. 4, no. 1, 1 janvier 2011 (2011-01-01) , pages 1-18, XP009172066, ISSN: 0974-3618
- Anonymous: "Handbook of Pharmaceutical Controlled Release Technology", 2000, Marcel dekker, Inc., USA, XP002715333, 3. Coating Technologies; page 473, alinéa 4

## Description

La présente invention porte sur une pastille pelliculée destinée à se dissoudre dans la cavité buccale et permettant l'administration d'un principe actif par voie buccale ainsi que sur son utilisation en tant que médicament.

Les formes galéniques permettant l'administration de principe actif par voie buccale sont particulièrement utiles pour l'administration de principes actifs susceptibles de se dégrader lors de l'ingestion et/ou susceptibles de subir un effet de premier passage hépatique mais pouvant pénétrer dans la circulation sanguine en passant à travers la muqueuse buccale.

La nicotine est une substance hautement addictive présente dans la cigarette. La plupart des fumeurs éprouvent des difficultés à s'abstenir ou s'arrêter de fumer car ils éprouvent rapidement des sensations de manque. L'apport de nicotine par un substitut à la cigarette permet de combler ce manque et donc de favoriser l'arrêt du tabac.

Ces substituts peuvent se présenter sous différentes formes galéniques notamment les gommes à mâcher, les comprimés sublinguaux, les inhalateurs, les comprimés à sucer ou les pastilles à sucer.

Les gommes à mâcher, les comprimés à sucer et les pastilles à sucer ont pour avantage, outre l'apport de nicotine, de distraire l'attention du patient en arrêt de cigarette par une action physique : mâcher ou sucer ce qui favorise l'oubli de l'envie de cigarette.

US2004/101543 divulgue des formes galéniques orales solides comprenant de la nicotine, qui sont utiles pour réduire ou prévenir les envies de nicotine par administration transmuqueuse orale de nicotine. Les formes galéniques comprennent une matrice vitreuse comprenant au moins un alcool de sucre sensiblement non hygroscopique et de la nicotine.

Les gommes à mâcher (telles que notamment décrites dans le brevet US3877468) présentent un certain nombre de désavantages, en particulier, en ce qui concerne la dose de nicotine assimilée. En effet, il est difficile de réguler la quantité totale de nicotine libérée par un chewing-gum ainsi que sa cinétique de libération, celles-ci étant fortement influencées par l'utilisation qu'en fait le patient. Les indications de mâchage (quelques mastications puis arrêt et maintien de la gomme contre la joue) sont rarement respectées par les patients. Ceux-ci ont, au contraire, tendance à mâcher fortement la gomme en début d'utilisation au moment où l'envie de fumer est la plus forte. Ceci résulte en une libération trop rapide de la nicotine, un trop grand passage de la nicotine par la voie digestive (ou elle sera majoritairement dégradée) et donc un sous dosage de la nicotine dans la gomme restante. Cette ingestion trop rapide de nicotine engendre en outre de nombreux effets secondaires (nausée, douleur d'estomac, goût désagréable). Le mâchage est en outre inesthétique et délétère vis à vis des prothèses dentaires.

Les pastilles à sucer à base de sucre cuit, notamment vendues sous la marque Nicopass^{®} (voir en particulier WO2002/085334), permettent d'éviter les problèmes liés aux gommes à mâcher notamment en délivrant de façon régulière et totale la nicotine pendant environ 30 minutes, sans possibilité pour l'utilisateur d'accélérer la fonte de la pastille.

Ces pastilles peuvent cependant ne pas être totalement adaptées à de gros fumeurs ou des fumeurs fortement dépendants. En effet, chez ce genre de patients, l'envie de fumer est particulièrement forte pendant les premières minutes et décroit ensuite peu à peu. Les pastilles à sucer actuellement disponibles sur le commerce peuvent être considérées comme trop lentes à combler l'envie première de cigarette chez ces patients.

Il existe donc de nos jours un réel besoin d'un substitut nicotinique alternatif permettant la diffusion régulière de nicotine pendant un temps suffisamment long pour que l'envie de fumer ait totalement disparue et de façon suffisamment rapide pour compenser l'envie de fumer précoce que connaissent les gros fumeurs ou les fumeurs fortement dépendants. Il est, en outre, préférable pour un bon suivi du traitement que le substitut soit agréable au goût et qu'il n'engendre pas ou peu d'effets secondaires.

### FIGURES :

Figure 1 : Vue schématique d'une coupe latérale d'une pastille selon l'invention comprenant un noyau, une enveloppe constituée d'une couche isolante interne, d'une couche comprenant au moins un principe actif et d'une couche isolante externe ainsi que d'une couche externe enrobant l'enveloppe.
Figure 2 : Vue schématique d'une demi-coupe latérale d'une pastille hors invention comprenant un noyau, une enveloppe constituée d'une alternance de couches isolantes et de couches comprenant au moins un principe actif ainsi que d'une couche externe enrobant l'enveloppe.

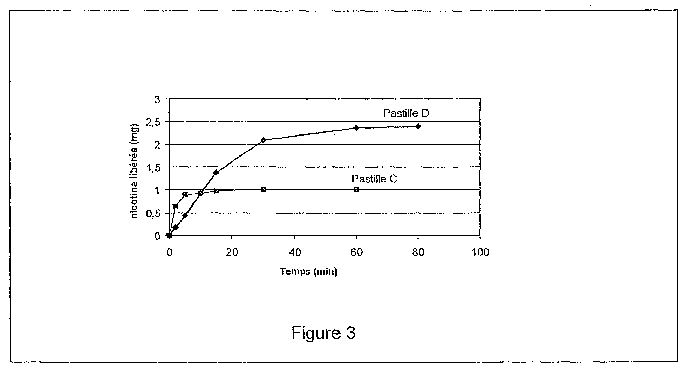

Figure 3 : Cinétique de libération de la nicotine d'une pastille C (carré) et D (losange).

De façon surprenante dans la présente invention, il a été mis au point une nouvelle forme galénique destinée à se dissoudre dans la cavité buccale permettant l'administration d'un principe actif selon une double cinétique : rapide dans les toutes premières minutes de la prise par le patient puis prolongée jusqu'à la fin de la sensation de manque.

La présente invention porte donc sur une pastille pelliculée destinée à se dissoudre dans la cavité buccale comprenant :
- un noyau comprenant au moins un principe actif et au moins un sucre cuit,
- une enveloppe,
   caractérisée en ce que l'enveloppe consiste successivement en une couche isolante interne comprenant au moins un polymère filmogène, une couche comprenant au moins un principe actif et une couche isolante externe comprenant au moins un polymère filmogène,
   et dans laquelle le noyau et la couche de l'enveloppe comprenant au moins un principe actif comprennent respectivement un seul principe actif, le principe actif étant respectivement le résinate de nicotine dans le noyau et le bitartrate de nicotine dans la couche de l'enveloppe comprenant un principe actif,
   caractérisée en outre en ce que :
      la pastille comprend en outre une couche externe enrobant l'enveloppe comprenant un polymère filmogène,
      le temps de dissolution de la couche externe enrobant l'enveloppe et de l'enveloppe, tel que mesuré par un test de dissolution dans un tampon à pH physiologique tel que décrit à l'exemple 3, est compris entre 5 secondes et 5 minutes,
      le temps de dissolution total de la pastille pelliculée est compris entre 15 et 40 minutes, mesuré par un test de dissolution dans un tampon à pH physiologique tel que décrit à l'exemple 3, et
      le polymère filmogène est la copovidone.

Par « pastille » selon l'invention on entend une forme galénique bien spécifique, ayant notamment fait l'objet d'une monographie à la pharmacopée Française Xème édition Quillet 1987). Les pastilles sont des saccharoïdes de consistance solide et dure obtenues par une opération de cuisson d'un polyol et destinées à se dissoudre lentement dans la cavité buccale. Les pastilles selon l'invention sont des pastilles enrobées ou pelliculées qui ont préférentiellement une forme ronde et plate ou une forme de bille. Le terme pastille selon l'invention correspond aux termes anglais « lozenge » ou « hard candy ». Par pelliculée on entend que la pastille selon l'invention est enrobée d'une enveloppe et d'une couche externe. L'enveloppe et la couche externe seront préférentiellement fines c'est à dire que leur épaisseur est inférieure à 1 mm, préférentiellement inférieure à 0,1 mm, préférentiellement comprise entre 1 µm et 1mm, encore préférentiellement entre 10 µm et 0,1 mm.

Par « destinée à se dissoudre dans la cavité buccale » on entend que la pastille selon l'invention est capable de se dissoudre dans la bouche au contact de la salive. La pastille selon l'invention permet le relargage de la totalité du principe actif qu'elle contient dans la cavité buccale. La pastille selon l'invention aura donc une dissolution uniquement buccale et ne sera pas destinée à être avalée. Préférentiellement, le noyau de la pastille selon l'invention sera suffisamment résistant pour qu'il ne soit pas possible ou difficile pour le patient de croquer cette pastille et donc d'en avaler des morceaux (la résistance de la pastille pourra être fonction de sa dureté, par exemple la pastille selon l'invention pourra avoir une dureté comprise entre 300 et 700N, par exemple entre 400 et 600N telle que mesurée par un duromètre type Schleuniger. Le patient est alors obligé de sucer la pastille jusqu'à sa disparition ce qui assure l'apport constant et régulier de nicotine.

Dans un mode de réalisation, le noyau de la pastille selon l'invention aura un temps de dissolution total compris entre 15 et 35 minutes, préférentiellement entre 15 et 30 minutes, encore plus préférentiellement entre 25 et 30 minutes, par exemple 30 minutes.

La pastille selon l'invention aura un temps de dissolution total compris entre 15 et 40 minutes, préférentiellement entre 15 et 30 minutes, encore plus préférentiellement entre 25 et 30 minutes, par exemple 30 minutes.

Le noyau de la pastille selon l'invention comprend au moins un sucre cuit. Par sucre cuit on entend un sucre obtenu par cuisson d'un polyol ou ose.

Le polyol sera préférentiellement sélectionné dans le groupe comprenant ou consistant en le saccharose, le fructose, le lactose, le sorbitol, le mannitol, le lactitol, le glucose, l'isomalt, le polydextrose et les maltodextrines ou leurs mélanges, préférentiellement l'isomalt. Le polyol selon l'invention représentera de préférence 80 à 97% en poids de la pastille, préférentiellement entre 85 et 95%, par exemple autour de 90%.

Les techniques d'obtention d'un sucre cuit selon l'invention sont bien connues de l'homme du métier et sont notamment décrites dans « sugar confectionery and manufacture » (E.B.Jackson (Springer, 1995)). WO2002/085334 divulgue également la fabrication de ce type de formulation, donc apte à être appliquée à la présente invention. Les conditions choisies pour obtenir le sucre cuit pourront dépendre de plusieurs paramètres notamment la température, la pression, la concentration en sucre et en eau. Par exemple, le sucre selon l'invention pourra être cuit à une température comprise entre 140 et 200°C, par exemple entre 150 et 180°C à pression atmosphérique. Le sucre après cuisson est dans un état vitreux.

Afin d'augmenter sa résistance le noyau de la pastille selon l'invention pourra contenir un agent matriciel. Les agents matriciels préférés sont préférentiellement sélectionnés dans le groupe comprenant ou consistant en les polysaccharides non cellulosiques, les dérivés cellulosiques, les polymères de l'acide acrylique, les corps gras, le polyvinylpyrrolidone ou leur mélange, préférentiellement le polyvinylpyrrolidone. Préférentiellement, l'agent matriciel sera sélectionné parmi ceux décrits dans la demande de brevet WO2002/085334. Notamment l'agent matriciel pourra être typiquement choisi dans le groupe comprenant ou consistant en les polysaccharides non cellulosiques, les dérivés cellulosiques (notamment l'hydroxypropylcellulose, l'hydroxypropylméthylcellulose, l'hydroxyéthylcellulose, l'éthylcellulose), les polymères de l'acide acrylique, les corps gras, le polyvinylpyrrolidone, les gommes ou leurs mélanges. Dans un mode de réalisation, l'agent matriciel représente entre 1 à 10% en poids de la pastille, typiquement 1 à 5%.

Le noyau de la pastille selon l'invention pourra en outre comprendre au moins une substance auxiliaire choisie notamment parmi les édulcorants, les agents tampons, les antioxydants, les colorants ou les arômes. Le noyau de la pastille est destiné à être dur pour pouvoir être sucé. Le noyau ne comprendra donc préférentiellement peu ou pas de matières telle que gomme, pectines et autres agents texturant rendant la pastille insuffisamment dure du type « gomme à mâcher ».

Dans des modes de réalisation de l'invention, le noyau de la pastille sera tel que défini dans la demande WO2002/085334 (décrit sous le terme de « pastille ») et notamment tel que décrit dans les exemples 1 à 9 de la demande WO2002/085334, ou dans les exemples de la présente demande.

Par « couche interne » selon l'invention, on entend une couche plus proche séquentiellement du noyau vis-à-vis d'une « couche externe ».

Par « couche externe » selon l'invention, on entend une couche plus éloignée séquentiellement du noyau vis-à-vis d'une « couche interne ».

Par « séquentiellement », on entend le passage d'une couche à une autre, à la manière d'une séquence de couches.

Dans un mode de réalisation préféré, les couches isolantes ne comprennent pas de principe actif.

L'enveloppe selon l'invention comprend plus d'une couche isolante. On nommera « couche isolante interne » la couche isolante la plus proche séquentiellement du noyau vis-à-vis de la « couche isolante externe » la couche isolante la plus éloignée séquentiellement du noyau vis-à-vis de la « couche isolante interne ». Selon l'invention, l'enveloppe comprend une couche isolante interne et une couche isolante externe.

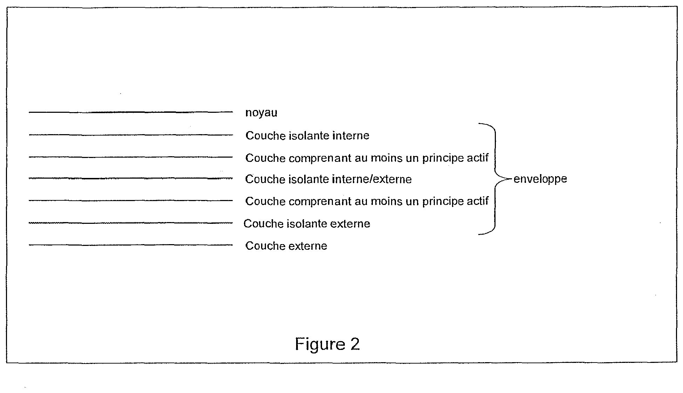

Dans un mode de réalisation (non couvert par l'invention), l'enveloppe comprend une alternance de couches isolantes et de couches comprenant au moins un principe actif. Un exemple de ce mode de réalisation est illustré à la figure 2. On peut noter que dans le mode de réalisation illustré à la figure 2, la couche isolante dans l'enveloppe localisée entre deux couches comprenant au moins un principe actif est à la fois couche isolante interne et externe.

Selon l'invention, l'enveloppe consiste en une couche isolante interne, une couche comprenant un seul principe actif et une couche isolante externe.

Selon l'invention, l'enveloppe comprend uniquement deux couches isolantes. Ces couches seront une couche isolante interne dans l'enveloppe localisée entre le noyau et la couche de l'enveloppe comprenant le principe actif et une couche isolante externe dans l'enveloppe localisée après la couche comprenant le principe actif la plus éloignée du noyau.

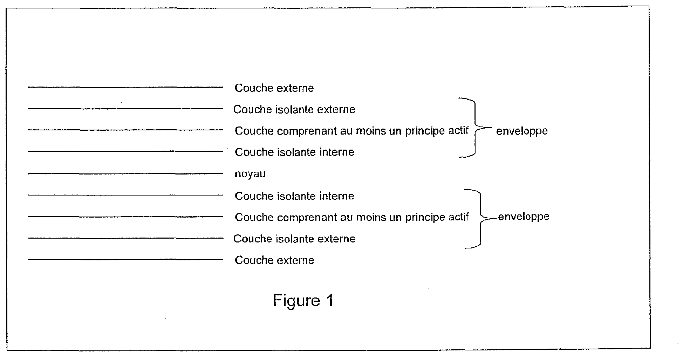

La pastille selon l'invention, illustrée dans la figure 1, consiste en :
- un noyau,
- une enveloppe consistant en successivement :
   - une couche isolante interne,
   - une couche comprenant au moins un principe actif,
   - une couche isolante externe, et
- une couche externe enrobant l'enveloppe.

Le noyau et la couche de l'enveloppe comprenant au moins un principe actif comprennent chacun un seul principe actif. Selon l'invention, le sel de nicotine compris dans le noyau est le résinate de nicotine et le sel compris dans la couche de l'enveloppe comprenant le principe actif est le bitartrate de nicotine.

Les inventeurs ont mis en évidence qu'il était hautement avantageux de séparer par des couches isolantes la couche comprenant au moins un principe actif, du noyau, et éventuellement de la couche externe enrobant le noyau et/ou des autres couches comprenant au moins un principe actif. En effet, la présence de ces couches isolantes permet d'éviter des interactions entre les composés/excipients des différentes couches adjacentes entres elles ou avec le noyau. Lorsque le principe actif contenu dans la couche de l'enveloppe comprenant au moins un principe actif est un sel de nicotine, les couches isolantes dans l'enveloppe selon l'invention ont pour avantage d'isoler ce sel de nicotine de l'agent tampon éventuellement contenu dans le noyau et/ou dans la couche externe enrobant l'enveloppe. En effet, les inventeurs ont constaté que le contact entre le sel nicotine et l'agent tampon facilite la désalification du sel de nicotine et la formation de la nicotine base beaucoup plus volatile que le sel. La présence d'une couche isolante dans l'enveloppe permet donc de limiter cette transformation et donc de limiter l'évaporation de la nicotine du film à dissolution rapide.

La présence d'une couche isolante adjacente au noyau a par ailleurs comme avantage de faciliter la production de la pastille pelliculée selon l'invention en favorisant l'adhésion des couches suivantes.

Une couche isolante selon l'invention comprend au moins un polymère filmogène.

Les inventeurs ont par ailleurs mis en évidence qu'il pouvait être hautement avantageux de sélectionner des sels de nicotine différents pour le noyau et le l'enveloppe/pelliculage de la pastille selon l'invention. En particulier, l'utilisation du résinate de nicotine dans le noyau permet une bonne résistance, en particulier, à la chaleur du principe actif. Le ditartrate de nicotine à quant à lui été identifié comme étant particulièrement adapté au pelliculage/enveloppe car il permet une mise à disposition rapide de l'actif et est, par ailleurs, particulièrement facile à mettre en solution lors des étapes de fabrication de la pastille.

Préférentiellement, la quantité de nicotine base ou en équivalent base comprise dans la couche de l'enveloppe comprenant au moins un principe actif selon l'invention sera comprise entre 9 et 50% en poids de la quantité totale de nicotine base ou en équivalent base comprise dans la pastille pelliculée selon l'invention, par exemple entre 15 et 40%, encore par exemple autour ou de 30%. Par exemple le noyau selon l'invention comprendra entre 1 et 5 mg de nicotine pure, par exemple 1mg, 2mg, 2,5 mg ou 4mg et la couche de l'enveloppe comprenant le principe actif comprendra entre 0,1 et 2 mg de nicotine pure, par exemple 0,5 mg, 1 mg ou 1,5 mg de nicotine pure. Dans un mode de réalisation la quantité totale de nicotine présente dans la pastille selon l'invention sera comprise entre 3 et 5 mg, par exemple 4 mg.

La pastille pelliculée selon l'invention comprend en outre une couche externe. Par « couche externe » ou « couche externe enrobant l'enveloppe » selon l'invention on entend une couche enrobant l'extérieur de l'enveloppe selon l'invention. Cette couche est donc directement en contact avec le milieu environnant de la pastille. La présence d'une couche externe enrobant l'enveloppe est particulièrement envisagée lorsque la pastille pelliculée selon l'invention comprend un ou des principes actifs sensibles au contact avec l'air.

Par « enrobant » selon l'invention on entend que ladite enveloppe recouvre totalement la surface du noyau et la couche externe recouvre totalement la surface de l'enveloppe. Par totalement, on comprend selon l'invention que le recouvrement est de plus de 95%, préférentiellement de plus de 99%, par exemple de 100% de la surface du noyau ou de l'enveloppe respectivement.

La couche de l'enveloppe comprenant au moins un principe actif comprend préférentiellement au moins un, préférentiellement un, polymère à délitement rapide ou filmogène. Dans un mode de réalisation le ou les polymères présents dans l'enveloppe, dans la ou les couches comprenant au moins un principe actif, et la ou les couches isolantes (externes ou internes) et/ou la couche externe sont identiques.

Le polymère à délitement rapide ou filmogène selon l'invention est préférentiellement sélectionné dans le groupe comprenant ou consistant en la copovidone, la vinylpyrrolidone, le vinyl acétate, l'hydroxypropylcellulose, l'hydroxypropylméthylcellulose, la polyvinylpyrrolidone, le macrogol, le polyvinylalcool, l'acide méthacrilique, l'éthyle acrylate co-polymère (dans un rapport de 1 :1 - 1 :2), l'hypromellose, la gomme shellac, l'hydroxyéthylcellulose, le copolymère d'amonio méthacrylate (type A et B), le copolymère d'acide méthacrilique- méthyl méthacrylate (dans un rapport de 1 :1 - 1 :2), le polyacrylate dispersion 30%, préférentiellement la copovidone.

La couche externe enrobant l'enveloppe pourra en outre comprendre un ou plusieurs agents filmogènes, agents plastifiants, agents opacifiants, agents anti-agglomérants, colorants, édulcorants, agents tampons, arômes. Préférentiellement, la couche externe ne comprend pas de principe actif.

La quantité de polymère à délitement rapide ou filmogène présent dans la couche de l'enveloppe comprenant au moins un principe actif, la couche externe enrobant l'enveloppe et la/les couches isolantes dans l'enveloppe sera préférentiellement comprise entre 55% et 75%, préférentiellement entre 60 et 70%, par exemple autour de 65%, de la quantité totale de matière sèche contenue dans chacune des différentes couches. Ces pourcentages permettent notamment pour les couches isolantes d'assurer une bonne isolation entre les couches entres elles ou avec le noyau qu'elles séparent et donc d'éviter les interactions entre celles-ci.

La couche externe enrobant l'enveloppe comprend préférentiellement un agent tampon. Préférentiellement, l'agent tampon compris dans la couche externe sera tel qu'il permet lors de la dissolution de la couche externe d'alcaliniser momentanément le pH de la cavité buccale entre 8 et 10,5, par exemple entre 9 et 10, afin de favoriser la formation de la nicotine base à partir du sel de nicotine contenu dans la pastille selon l'invention.

A titre d'agent plastifiant selon l'invention on peut notamment citer le macrogol, le glycérol, le triéthyle citrate, le dibutyle sébaçate, la triacétine, le propylène glycol et le triéthyle acétate. Dans un mode de réalisation l'agent plastifiant sera le macrogol, le glycérol et/ou le propylène glycol. Ces agents plastifiants ont en particulier l'avantage de ne pas apporter d'amertume.

A titre d'agent opacifiant selon l'invention on peut notamment citer le dioxyde de titane.

A titre d'agent anti-agglomérant selon l'invention on peut notamment citer le talc.

Le colorant selon l'invention peut être n'importe quel colorant sous forme soluble ou insoluble notamment la laque ou l'oxyde de fer.

A titre d'édulcorant selon l'invention on peut notamment citer la saccharine sodique, la saccharine acide, l'aspartame, l'acesulfame potassique, le cyclamate de sodium, le sucralose, le néotame, l'alitame, les dérivés de la stévia (par exemple la *stévia eupatoria* ou la *stévia rebaudiana*). Dans un mode de réalisation l'édulcorant selon l'invention sera l'acésulfame potassique ou un mélange d'acésulfame potassique et de saccharine sodique.

A titre d'agent tampon selon l'invention on peut notamment citer le carbonate de calcium, le phosphate de sodium, le silicate de sodium, le citrate de sodium, l'hydroxyde de sodium, l'hydroxyde de potassium, le carbonate de sodium, le bicarbonate de sodium, le citrate de potassium, l'acide malique, le phosphate disodique, l'acide ascorbique, l'acide citrique, le lactate de sodium, l'acide tartrique, le phosphate tétrapotassique, le phosphate trisodique, préférentiellement le bicarbonate de sodium et le carbonate de sodium. Préférentiellement, le noyau selon l'invention comprendra entre 0 et 5% d'agent tampon, encore préférentiellement entre 0,5 et 2% d'agent tampon. Préférentiellement, la couche externe selon l'invention comprendra entre 5 et 20% d'agent tampon, préférentiellement entre 8 et 15% d'agent tampon.

A titre d'arôme on peut notamment citer tous les arômes utilisables dans les pastilles, notes de fruits (y compris agrumes), notes de fleurs, notes de baies réglisse, anis, café chocolat, vanille, caramel, menthe ainsi que les arômes dits « masqueurs de goût ». Dans un mode de réalisation, l'arôme sera sélectionné parmi les arômes se fixant aux mêmes récepteurs gustatifs que la nicotine tel que l'arôme menthe par exemple. Lorsque le principe actif est la nicotine la présence d'un arôme est préférée. En effet, les inventeurs ont constaté de façon surprenante que la présence d'un arôme dans la couche externe enrobant l'enveloppe était suffisant pour masquer en grande partie le goût de la nicotine libérée par l'enveloppe et ce malgré la rapidité de la dissolution l'enveloppe. Dans un mode de réalisation le noyau et la couche externe enrobant l'enveloppe comprennent au moins un arôme, préférentiellement le même arôme, par exemple la menthe. L'arôme pourra par exemple être présent entre 95% à 98% dans le noyau et entre 2% à 5% dans la couche externe enrobant l'enveloppe.

A titre d'agent antioxydant selon l'invention on peut notamment l'acide ascorbique, l'acide citrique monohydraté, l'acide fumarique, l'acide malique, l'acide araboascorbique, la méthionine, l'ascorbate de sodium, le sulfite et métabisulfite de sodium, l'acide tartarique.

Lorsque la pastille pelliculée selon l'invention comprend un agent antioxydant celui-ci pourra être présent dans une proportion allant de 0,01 % à 1 %.

Dans un mode de réalisation, la pastille selon l'invention, en particulier la pastille selon l'invention comprenant de la nicotine en tant que principe actif ne comprend pas d'agent antioxydant.

Le temps de dissolution de la couche externe enrobant l'enveloppe et de l'enveloppe est compris entre 5 secondes et 5 minutes, préférentiellement inférieur à 3 minutes, encore préférentiellement inférieur à 1 minute ou à 30 secondes.

Le temps de dissolution correspond au temps de libération d'au moins 70%, préférentiellement d'au moins 90%, du ou des principes actifs dans les couches envisagées. La vitesse de dissolution de la couche externe enrobant l'enveloppe et de l'enveloppe rapide peut notamment être mesurée par un test de dissolution dans du tampon à pH physiologique comme notamment décrit dans l'exemple 3.

La présence d'une enveloppe et d'une couche externe enrobant l'enveloppe augmente la vitesse de libération de la nicotine par rapport à la vitesse de libération de la nicotine à partir du noyau seul, permettant l'apparition de nicotine plus rapidement dans l'organisme, sans toutefois modifier le Cmax ou le Tmax de la nicotine. Le Tmax et le Cmax peuvent facilement être déterminés par l'homme du métier par mesure de la quantité de nicotine dans le sang ou le plasma en fonction du temps après la prise de la pastille selon l'invention. On considérera que le Cmax de la cinétique de nicotine n'est pas modifié par la présence de l'enveloppe et/ou de la couche externe enrobant l'enveloppe par rapport au noyau seul si les valeurs de Cmax ne varient pas de plus de 20%.

Dans un mode de réalisation particulier, la pastille pelliculée selon la présente invention comprend ou consiste en :
- un noyau comprenant ou consistant en 0,1 à 10% en poids sec de résinate de nicotine, de 70 à 95% en poids sec d'isomalt, et avantageusement de 0,1 à 10% en poids sec d'hypromellose, de 0 à 2% en poids sec d'aspartame, de 0 à 2% en poids sec d'acésulfame potassique, de 0 à 5% en poids sec d'arômes , par exemple « Peppermint » (menthe poivrée) et/ou de masqueurs de goût, de 0 à 2% en poids sec de bicarbonate de sodium, de 0 à 4% en poids sec de carbonate de sodium anhydre, par rapport au poids sec total du noyau ;
- une enveloppe consistant en :
   - une couche isolante interne comprenant de 10 à 90% en poids sec de copovidone et avantageusement de 10 à 90% en poids sec de talc (qualité pharmaceutique), par rapport au poids sec total de ladite couche isolante interne ;
   - d'une couche comprenant un principe actif comprenant ou consistant en 0,1 à 10% en poids sec de dihydrate de ditartrate de nicotine, de 5 à 90% en poids sec de copovidone et de 10 à 90% en poids sec de talc (qualité pharmaceutique), par rapport au poids sec total de ladite couche comprenant un principe actif ;
   - une couche isolante externe comprenant ou consistant en 10 à 90% en poids sec de copovidone et avantageusement de 10 à 90% en poids sec de talc (qualité pharmaceutique), par rapport au poids sec total de ladite couche isolante interne ;
- une couche externe enrobant l'enveloppe comprenant ou consistant en 10 à 90% en poids sec de copovidone et avantageusement de 10 à 90% en poids sec de talc (qualité pharmaceutique), de 0 à 30% en poids sec de dioxyde de titane, de 0 à 30% en poids sec d'acésulfame potassique, de 0 à 10% en poids sec de colorant, de 0 à 10% en poids sec de carbonate de sodium anhydre, de 0 à 20% en poids sec de bicarbonate de sodium, de 0 à 20% en poids sec d'arôme, par rapport au poids sec total de ladite couche externe.

Préférentiellement, la pastille pelliculée selon la présente invention comprend ou consiste en :
- un noyau comprenant ou consistant en 0,2 à 1% en poids sec de résinate de nicotine, de 90 à 95% en poids sec d'isomalt, et avantageusement de 1 à 5% en poids sec d'hypromellose, de 0,01 à 0,5% en poids sec d'aspartame, de 0,01 à 0,5% en poids sec d'acésulfame potassique, de 0,5 à 2% en poids sec d'arômes , par exemple « Peppermint » (menthe poivrée) et/ou de masqueurs de goût, de 0,1 à 1% en poids sec de bicarbonate de sodium, de 0,5 à 1% en poids sec de carbonate de sodium anhydre, par rapport au poids sec total du noyau ;
- une enveloppe consistant en :
   - une couche isolante interne comprenant ou consistant en 50 à 80% en poids sec de copovidone et avantageusement de 20 à 50% en poids sec de talc (qualité pharmaceutique), par rapport au poids sec total de ladite couche isolante interne ;
   - d'une couche comprenant un principe actif comprenant ou consistant en 1 à 5% en poids sec de dihydrate de ditartrate de nicotine, de 40 à 80% en poids sec de copovidone et avantageusement de 19 à 55% en poids sec de talc (qualité pharmaceutique), par rapport au poids sec total de ladite couche comprenant un principe actif ;
   - une éventuelle couche isolante externe comprenant ou consistant en 50 à 80% en poids sec de copovidone et avantageusement de 20 à 50% en poids sec de talc (qualité pharmaceutique), par rapport au poids sec total de ladite couche isolante interne ;
- une couche externe enrobant l'enveloppe comprenant ou consistant en 20 à 70% en poids sec de copovidone et avantageusement de 10 à 50% en poids sec de talc (qualité pharmaceutique), de 5 à 15% en poids sec de dioxyde de titane, de 1 à 10% en poids sec d'acésulfame potassique, de 2 à 7% en poids sec de colorant, de 5 à 10% en poids sec de carbonate de sodium anhydre, de 1 à 10% en poids sec de bicarbonate de sodium, de 0 à 10% en poids sec d'arôme, par rapport au poids sec total de ladite couche externe.

Plus préférentiellement, la pastille pelliculée selon la présente invention comprend ou consiste en :
- un noyau comprenant ou consistant en 0,4 à 0,7% en poids sec de résinate de nicotine, de 92 à 93% en poids sec d'isomalt, et avantageusement de 3 à 5% en poids sec d'hypromellose, de 0,03 à 0,05% en poids sec d'aspartame, de 0,05 à 0,07% en poids sec d'acésulfame potassique, de 1,5 à 1,6% en poids sec d'arômes, par exemple « Peppermint » (menthe poivrée) et/ou de masqueurs de goût, de 0,4 à 0,5% en poids sec de bicarbonate de sodium, de 0,9 à 1% en poids sec de carbonate de sodium anhydre, par rapport au poids sec total du noyau ;
- une enveloppe consistant en :
   - une couche isolante interne comprenant ou consistant en 60 à 70% en poids sec de copovidone et avantageusement de 30 à 40% en poids sec de talc (qualité pharmaceutique), par rapport au poids sec total de ladite couche isolante interne ;
   - d'une couche comprenant un principe actif comprenant ou consistant en 3 à 5% en poids sec de dihydrate de ditartrate de nicotine, et avantageusement de 70 à 80% en poids sec de copovidone et de 20 à 30% en poids sec de talc (qualité pharmaceutique), par rapport au poids sec total de ladite couche comprenant un principe actif ;
   - une couche isolante externe comprenant ou consistant en 60 à 75% en poids sec de copovidone et avantageusement de 25 à 40% en poids sec de talc (qualité pharmaceutique), par rapport au poids sec total de ladite couche isolante interne ;
- une couche externe comprenant ou consistant en 40 à 50% en poids sec de copovidone et avantageusement de 10 à 20% en poids sec de talc (qualité pharmaceutique), de 8 à 12% en poids sec de dioxyde de titane, de 1,5 à 3,5% en poids sec d'acésulfame potassique, de 4 à 6% en poids sec de colorant, de 6 à 8% en poids sec de carbonate de sodium anhydre, de 3 à 4,5% en poids sec de bicarbonate de sodium, de 1 à 3% en poids sec d'arôme, par rapport au poids sec total de ladite couche externe.

La présente invention porte, en outre, sur un procédé de préparation d'une pastille pelliculée selon l'invention comprenant ou consistant en les étapes successives suivantes :
a. préparation d'un noyau comprenant au moins un principe actif et au moins un sucre cuit selon l'invention,
b. pulvérisation puis séchage d'une enveloppe selon l'invention sur le noyau obtenu à l'étape a, et
c. pulvérisation puis séchage d'une couche externe selon l'invention sur le noyau enrobé obtenu à l'étape b.

Dans un mode de réalisation, dans le procédé selon l'invention l'étape b peut comprendre ou consister en les étapes successives suivantes :
b1. pulvérisation puis séchage d'au moins une couche isolante interne selon l'invention,
b2. pulvérisation puis séchage d'au moins une couche comprenant au moins un principe actif selon l'invention,
b3. pulvérisation puis séchage d'au moins une couche isolante selon l'invention, préférentiellement une couche isolante externe.

La présente invention porte, en outre, sur une pastille pelliculée selon l'invention pour son utilisation en tant que médicament.

Dans un mode de réalisation, la présente invention porte sur une pastille selon l'invention, en particulier une pastille comprenant de la nicotine ou un de ses sels ou ses complexes pour son utilisation dans le traitement de la dépendance tabagique.

### EXEMPLES

### Exemple 1 : Obtention d'une pastille comprenant un noyau, une enveloppe constituée d'une couche isolante interne, d'une couche comprenant de la nicotine et d'une couche isolante externe ainsi qu'une couche externe

### 1. Obtention du noyau de la pastille :

### 2.1. Composition du noyau de la pastille A :

Un noyau comprenant les constituants décrits dans le tableau 1 est préparé à chaud avec une quantité suffisante d'eau pour la réalisation du procédé. L'ensemble des éléments, dont l'agent matriciel, l'eau et la base sucrante, à l'exception de la nicotine sont mélangés à une température de 90°C (la température peut varier entre 70 et 95°C, température d'ébullition). L'ensemble est ensuite chauffé à 150°C au moment de la cuisson. La nicotine est alors ajoutée dans la masse à une température de 120°C (la température peut varier entre 110 et 130°C).

**Tableau 1 : composition du noyau de la pastille A**

| **Composants** | **Quantités (mg) (pastilles sèches)** | **Pourcentage sec** |
|---|---|---|
| Nicotine Résinate 18% | 13,89 (soit 2,5 mg de nicotine pure) | 0,56% |
| Isomalt | 2310,36 | 92,41% |
| Hypromellose 6500 | 100 | 4% |
| Aspartam | 1 | 0,04% |
| Acésulfame potassique | 1,5 | 0,06% |
| Arômes et masqueur de goût | 38,25 | 1,53% |
| Bicarbonate de sodium | 11,67 | 0,47% |
| Carbonate de sodium anhydre | 23,33 | 0,93% |
| P.U.T | 2500 | 100% |

### 2. Préparation de la solution pour former la couche isolante de l'enveloppe:

De la copovidone (environ 8%) est solubilisée dans de l'eau purifiée. Environ 3,7% de Talc est ajoutée à la solution et mélangée par agitation. L'agitation de la solution sera maintenue pendant toute l'étape de pulvérisation (pourcentage en masse par rapport à la masse de la couche isolante).

### 3. Pulvérisation de la couche isolante interne de l'enveloppe :

Les noyaux de pastilles obtenus à l'étape 1 sont introduits dans une turbine de pelliculage perforée types Dumoulin, Driacoater, Simo, Accela Cota. La solution obtenue à l'étape 2 est pulvérisée sur les noyaux des pastilles en mouvement en réglant la température de l'air entrant de façon à extraire l'humidité engendrée et éviter de dénaturer le polyol contenu dans le noyau de la pastille. Cette température pourra être comprise entre 55°C et 65°C.

La pastille ainsi pelliculée est ensuite séchée à une température voisine de 40°C

### 4. Préparation de la solution pour former la couche de l'enveloppe comprenant le principe actif :

De la copovidone (environ 8%) est solubilisée dans de l'eau purifiée. Environ 2,5% de Talc et 1,5365 g de ditartrate de nicotine est ajoutée à la solution et mélangée par agitation. L'agitation de la solution sera maintenue pendant toute l'étape de pulvérisation (pourcentage en masse par rapport à la masse de la couche comprenant le principe actif).

### 5. Pulvérisation de la couche de l'enveloppe comprenant le principe actif :

Les noyaux de pastilles pelliculés par la couche isolante obtenus à l'étape 3 sont introduits dans une turbine de pelliculage perforée types Dumoulin, Driacoater, Simo, Accela Cota. La solution préparée à l'étape 4 est pulvérisée sur ces noyaux de pastilles pelliculés en mouvement en réglant la température de l'air entrant de façon à extraire l'humidité engendrée et éviter de dénaturer le polyol contenu dans le noyau de la pastille. Cette température pourra être comprise entre 55°C et 65°C.

La pastille ainsi pelliculée est ensuite séchée à une température voisine de 40°C.

### 6. Préparation de la solution pour former la couche isolante externe de l'enveloppe :

De la copovidone (environ 8,5%) est solubilisée dans de l'eau purifiée. Environ 3,7% de Talc est ajoutée à la solution et mélangée par agitation. L'agitation de la solution sera maintenue pendant toute l'étape de pulvérisation (pourcentage en masse par rapport à la masse de la couche isolante externe de l'enveloppe).

### 7. Pulvérisation de la couche isolante externe de l'enveloppe :

Les noyaux de pastilles pelliculés obtenus à l'étape 5 sont introduits dans une turbine de pelliculage perforée types Dumoulin, Driacoater, Simo, Accela Cota. La solution préparée à l'étape 4 est pulvérisée sur ces noyaux de pastilles pelliculés en mouvement en réglant la température de l'air entrant de façon à extraire l'humidité engendrée et éviter de dénaturer le polyol contenu dans le noyau de la pastille. Cette température pourra être comprise entre 55°C et 65°C.

La pastille ainsi pelliculée est ensuite séchée à une température voisine de 40°C.

### 8. Préparation de la solution pour former la couche externe enrobant l'enveloppe :

De la copovidone (environ 5,8%) est solubilisée dans de l'eau purifiée. 1,8% de Talc, 0,2% d'acesulfame potassique, 1,1% de dioxyde de titane, 0,6% de colorant, 0,85% de carbonate de sodium anhydre, 0,42% de bicarbonate de sodium et 0,22% d'arome sont ajoutés successivement sous agitation à la solution de copovidone jusqu'à complète solubilisation.

L'agitation de la solution sera maintenue pendant toute l'étape de pulvérisation.

### 9. Pulvérisation de la couche externe enrobant l'enveloppe :

Les noyaux de pastilles pelliculés obtenus à l'étape 7 sont introduits dans une turbine de pelliculage perforée types Dumoulin, Driacoater, Simo, Accela Cota. La solution préparée à l'étape 8 est pulvérisée sur ces noyaux de pastilles péliculés en mouvement en réglant la température de l'air entrant de façon à extraire l'humidité engendrée et éviter de dénaturer le polyol contenu dans le noyau de la pastille. Cette température pourra être comprise entre 55°C et 65°C.

La pastille ainsi pelliculée est ensuite séchée à une température voisine de 40°C.

Des pastilles contenant plusieurs couches comprenant au moins un principe actif et/ou plusieurs couches isolantes peuvent être obtenues en répétant les étapes 2 à 7 voulues après l'étape 7 et aussi souvent que le nombre de films à dissolution rapide souhaité.

### Exemple 2 : Pastille obtenue par le procédé de l'exemple 2

La pastille A pelliculée suivante à été obtenue suivant le procédé de l'exemple 1 :

### 2.1. Composition du noyau de la pastille A :

La composition du noyau est telle que décrite dans le tableau 1.

### 2.2. Composition du pelliculage de la pastille A :

**Tableau 2 : composition du pelliculage de la pastille A**

| | **Composants** | **Quantités (mg)** | **Pourcentage sec pour chaque couche** |
|---|---|---|---|
| **Couche isolante interne de l'enveloppe** | COPOVIDONE | 26 | 68,42% |
| | TALC PHARMA M | 12 | 31,58% |
| | EAU PURIFIEE | 290 | |
| **Couche de l'enveloppe comprenant le principe actif** | NICOTINE DITARTRATE DIHYDRATE | 1,5 | 4,23% |
| | COPOVIDONE | 26 | 73,24% |
| | TALC PHARMA M | 8 | 22,53% |
| | EAU PURIFIEE | 290 | |
| **Couche isolante externe de l'enveloppe** | COPOVIDONE | 30 | 69,77% |
| | TALC PHARMA M | 13 | 30,23% |
| | EAU PURIFIEE | 310 | |
| **Couche externe enrobant l'enveloppe** | COPOVIDONE | 26 | 52,60% |
| | TALC PHARMA M | 8 | 16,19% |
| | DIOXYDE DE TITANE | 5 | 10,12% |
| | ACESULFAME POTASSIQUE | 1 | 2,02% |
| | COLORANT | 2,715 | 5,49% |
| | CARBONATE DE SODIUM ANHYDRE | 3,81 | 7,71% |
| | BICARBONATE DE SODIUM | 1,904 | 3,85% |
| | AROME | 1 | 2,02% |
| | EAU PURIFIEE | 400 | |

Une pastille B pelliculée est obtenue de la même façon que la pastille A avec pour différence la présence de 1 mg de nicotine dans la couche de l'enveloppe comprenant le principe actif.

A titre de pastille comparative, une pastille C pelliculée est obtenue de la même façon que la pastille pelliculée B avec pour différence que le noyau de la pastille C ne contient pas de principe actif (afin d'obtenir des pastilles de même poids (2500 mg), la nicotine est remplacée par l'équivalent en poids en polyol).

A titre de pastille comparative, une pastille D sans pelliculage est obtenue qui consiste uniquement en le noyau de la pastille B.

### Exemple 3 : Cinétique de libération de la nicotine

Afin de mesurer le temps de libération de la nicotine par le pelliculage de la pastille selon l'invention, le temps de libération de la nicotine par la pastille C est comparé au temps de libération de la nicotine dans la pastille D.

Pour cela les pastilles C et D sont soumise à un test de dissolution dans un appareil à palette (Ph.Eur.), dans 500 ml de tampon phosphate à pH 7.0 (Ph.Eur.) et à une température 37°C (conditions mimant les conditions de dissolution buccale). Les pastilles sont agitées à 100 tours/min pendant 80 minutes. Du milieu de dissolution est prélevé à 2.5, 5.0, 10, 15, 30, 60 et 80 minutes et la nicotine contenue dans ce milieu est dosée.

La nicotine est dosée par chromatographie liquide à un débit de 1.0 ml/min (volume injecté : 50 µl) (Phase stationnaire : colonne Xbridge Shield RP18, longueur 150 mm, diamètre 4.6 mm, porosité 5.0 µm, température 50°C, Phase mobile : 970 ml KH2PO4 à 20 mM (ajusté à pH 6.5 avec NH4OH) + 30 ml acétonitrile) et détection UV à 254 nm.

L'expérience a été réalisée 6 fois. Les résultats moyens obtenus sont représentés dans la Figure 4.

Comme on peut le constater, la libération de la nicotine dans la pastille C est très rapide : elle commence instantanément, environ 70% de la nicotine est libérée au bout de 2,5 minutes et environ 90% de la nicotine est libérée au bout de 5 minutes.

Au contraire la libération de la nicotine par la pastille D non pelliculée commence plus lentement (environ 8% de la nicotine est libérée à 2,5 minutes et 16 % à 5 minutes) et se poursuit jusqu'à 60 minutes environ.

Ces résultats démontrent l'intérêt des pastilles selon l'invention qui combinent libération rapide de nicotine dans les premières minutes de dissolution et libération continue de nicotine pendant plus de 30 minutes.

## Revendications

1. Pastille pelliculée destinée à se dissoudre dans la cavité buccale comprenant :
- un noyau comprenant au moins un principe actif et au moins un sucre cuit, et
- une enveloppe,
**caractérisée en ce que** l'enveloppe consiste successivement en une couche isolante interne comprenant au moins un polymère filmogène, une couche comprenant au moins un principe actif et une couche isolante externe comprenant au moins un polymère filmogène,
et dans laquelle le noyau et la couche de l'enveloppe comprenant au moins un principe actif comprennent respectivement un seul principe actif, le principe actif étant respectivement le résinate de nicotine dans le noyau et le bitartrate de nicotine dans la couche de l'enveloppe comprenant un principe actif,
**caractérisée en outre en ce que** :
la pastille comprend en outre une couche externe enrobant l'enveloppe comprenant un polymère filmogène,
le temps de dissolution de la couche externe enrobant l'enveloppe et de l'enveloppe, tel que mesuré par un test de dissolution dans un tampon à pH physiologique tel que décrit dans la description à l'exemple 3, est compris entre 5 secondes et 5 minutes
le temps de dissolution total de la pastille pelliculée est compris entre 15 et 40 minutes, mesuré par un test de dissolution dans un tampon à pH physiologique tel que décrit dans la description à l'exemple 3, et
le polymère filmogène est la copovidone.

2. Pastille pelliculée destinée à se dissoudre dans la cavité buccale selon la revendication 1, **caractérisée en ce que** la ou au moins une couche isolante est localisée entre le noyau et ladite au moins une couche comprenant le principe actif.

3. Pastille pelliculée selon l'une quelconque des revendications 1 à 2, dans laquelle l'enveloppe comprend au moins deux couches isolantes.

4. Pastille pelliculée selon l'une quelconque des revendications 1 à 3, dans laquelle la couche comprenant au moins un principe actif comprend un polymère filmogène.

5. Pastille pelliculée selon la revendication 4, dans laquelle le polymère filmogène est sélectionné dans le groupe comprenant la copovidone, la vinylpyrrolidone, le vinyl acétate, l'hydroxypropylcellulose, l'hydroxypropylméthylcellulose, la polyvinylpyrrolidone, le macrogol, le polyvinylalcool, l'acide méthacrilique, l'éthyl acrylate co-polymer (dans un rapport de 1 :1 - 1 :2), l'hypromellose, la gomme shellac, l'hydroxyéthylcellulose, le copolymère d'amonio méthacrilate (type A et B), le copolymère d'acide méthacrilique- méthyl méthacrylate (dans un rapport de 1 :1 - 1 :2) et le polyacrylate dispersion 30%.

6. Procédé de préparation d'une pastille pelliculée selon l'une quelconque des revendications 1 à 5, comprenant les étapes suivantes :
a. préparation d'un noyau comprenant au moins un principe actif et au moins un sucre cuit,
b. pulvérisation puis séchage d'au moins une enveloppe sur le noyau obtenu à l'étape a.
c. optionnellement, pulvérisation puis séchage d'une couche externe sur le noyau enrobé obtenu à l'étape b.

7. Procédé de préparation d'une pastille pelliculée selon la revendication 6, dans laquelle l'étape b. comprend les étapes successives suivantes :
b1. pulvérisation puis séchage d'au moins une couche isolante,
b2. pulvérisation puis séchage d'au moins une couche comprenant au moins un principe actif,
b3. pulvérisation puis séchage d'au moins une couche isolante,
les étapes b1 à b3 pouvant être répétées aussi souvent que nécessaire.

8. Pastille pelliculée selon l'une quelconque des revendications 1 à 5, pour son utilisation en tant que médicament.

9. Pastille pelliculée pour son utilisation selon la revendication 8, dans le traitement de la dépendance tabagique.

## Patentansprüche

1. Filmtablette zum Auflösen in der Mundhöhle, umfassend:
- einen Kern, der mindestens einen Wirkstoff und mindestens einen Backzucker enthält, und
- einen Mantel,
**dadurch gekennzeichnet, dass** der Mantel aufeinanderfolgend aus einer inneren Isolierschicht, die mindestens ein filmbildendes Polymer umfasst, einer Schicht, die mindestens einen Wirkstoff umfasst und einer äußeren Isolierschicht besteht, die mindestens ein filmbildendes Polymer umfasst,
und wobei der Kern und die Schicht des Mantels, die mindestens einen Wirkstoff umfasst, jeweils einen einzigen Wirkstoff enthalten, wobei der Wirkstoff jeweils Nikotinresinat im Kern und Nikotinbitartrat in der Schicht des Mantels, die einen Wirkstoff umfasst, ist,
ferner **dadurch gekennzeichnet, dass**:
die Tablette ferner eine äußere Schicht umfasst, die den Mantel umhüllt, die ein filmbildendes Polymer umfasst,
die Auflösungszeit der äußeren Schicht, die den Mantel umhüllt und des Mantels,
gemessen durch einen Auflösungstest in einem physiologischen pH-Puffer wie in der Beschreibung bei Beispiel 3 beschrieben, zwischen 5 Sekunden und 5 Minuten beträgt,
die Gesamtauflösungszeit der Filmtablette, gemessen durch einen Auflösungstest in einem physiologischen pH-Puffer wie in der Beschreibung bei Beispiel 3 beschrieben, zwischen 15 und 40 Minuten beträgt, und
das filmbildende Polymer Copovidon ist.

2. Filmtablette zum Auflösen in der Mundhöhle nach Anspruch 1, **dadurch gekennzeichnet, dass** sich die oder mindestens eine Isolierschicht zwischen dem Kern und der mindestens einen Schicht befindet, die den Wirkstoff umfasst.

3. Filmtablette nach einem der Ansprüche 1 bis 2, wobei der Mantel mindestens zwei Isolierschichten umfasst.

4. Filmtablette nach einem der Ansprüche 1 bis 3, wobei die Schicht, die mindestens einen Wirkstoff umfasst, ein filmbildendes Polymer umfasst.

5. Filmtablette nach Anspruch 4, wobei das filmbildende Polymer aus der Gruppe ausgewählt ist, die Copovidon, Vinylpyrrolidon, Vinylacetat, Hydroxypropylcellulose, Hydroxypropylmethylcellulose, Polyvinylpyrrolidon, Makrogol, Polyvinylalkohol, Methacrilsäure, Ethylacrylat-Copolymer (im Verhältnis von 1:1 - 1:2), Hypromellose, Shellac-Kautschuk, Hydroxyethylzellulose, Ammoniummethacrylat-Copolymer (Typ A und B), Methacrylsäure-Methylmethacrylat-Copolymer (im Verhältnis 1:1 - 1:2) und Polyacrylat-Dispersion 30 % umfasst.

6. Verfahren zur Herstellung einer Filmtablette nach einem der Ansprüche 1 bis 5, das die folgenden Schritte umfasst:
a. Herstellen eines Kerns, der mindestens einen Wirkstoff und mindestens einen Backzucker enthält,
b. Sprühen mindestens eines Mantels auf den in Schritt a. erhaltenen Kern und anschließendes Trocknen,
c. optional Sprühen einer äußeren Schicht auf den in Schritt b. umhüllten Kern und anschließendes Trocknen.

7. Verfahren zur Herstellung einer Filmtablette nach Anspruch 6, wobei Schritt b. die folgenden aufeinanderfolgenden Schritte umfasst:
b1. Sprühen und anschließendes Trocknen mindestens einer Isolierschicht,
b2. Sprühen und anschließendes Trocknen mindestens einer Schicht, die mindestens einen Wirkstoff umfasst,
b3. Sprühen und anschließendes Trocknen mindestens einer Isolierschicht, wobei die Schritte b1 bis b3 so oft wie nötig wiederholt werden können.

8. Filmtablette nach einem der Ansprüche 1 bis 5 zu ihrer Verwendung als Arzneimittel.

9. Filmtablette zu ihrer Verwendung nach Anspruch 8 bei der Behandlung der Tabakabhängigkeit.

## Claims

1. A film-coated lozenge intended to dissolve in the oral cavity comprising:
- a core comprising at least one active ingredient and at least one cooked sugar, and
- a shell,
**characterised in that** the shell consists successively of an inner insulating layer comprising at least one film forming polymer, a layer comprising at least one active ingredient and an outer insulating layer comprising at least one film forming polymer,
and wherein the core and the layer of the shell comprising at least one active ingredient comprise respectively a single active ingredient, the active ingredient being respectively nicotine resinate in the core and nicotine bitartrate in the layer of the shell comprising an active ingredient,
further **characterised in that**:
the lozenge further comprises an outer layer coating the shell comprising a film forming polymer,
the dissolution time of the outer layer coating the shell and of the shell, as measured by a dissolution test in a physiological pH buffer as described in the description in example 3, is between 5 seconds and 5 minutes the total dissolution time of the film-coated lozenge is between 15 and 40 minutes,
measured by a dissolution test in a physiological pH buffer as described in the description in example 3, and
the film forming polymer is copovidone.

2. The film-coated lozenge intended to dissolve in the oral cavity according to claim 1, **characterised in that** the or at least one insulating layer is located between the core and said at least one layer comprising the active ingredient.

3. The film-coated lozenge according to any of claims 1 to 2, wherein the shell comprises at least two insulating layers.

4. The film-coated lozenge according to any of claims 1 to 3, wherein the layer comprising at least one active ingredient comprises a film forming polymer.

5. The film-coated lozenge according to claim 4, wherein the film forming polymer is selected from the group comprising copovidone, vinylpyrrolidone, vinyl acetate, hydroxypropylcellulose, hydroxypropylmethylcellulose, polyvinylpyrrolidone, macrogol, polyvinylalcohol, methacrylic acid, ethyl acrylate co-polymer (in a ratio of 1:1 - 1:2), hypromellose, shellac gum, hydroxyethylcellulose, ammonio methacrylate copolymer (type A and B), methacrylic acid-methyl methacrylate copolymer (in a ratio of 1:1 - 1:2) and 30% dispersion polyacrylate.

6. A method for preparing a film-coated lozenge according to any of claims 1 to 5, comprising the following steps:
a. preparing a core comprising at least one active ingredient and at least one cooked sugar,
b. spraying and then drying at least one shell on the core obtained in step a.
c. optionally, spraying and then drying an outer layer on the coated core obtained in step b.

7. The method for preparing a film-coated lozenge according to claim 6, wherein step b. comprises the following successive steps of:
b1. spraying and then drying at least one insulating layer,
b2. spraying and then drying at least one layer comprising at least one active ingredient,
b3. spraying and then drying at least one insulating layer, steps b1 to b3 can be repeated as often as necessary.

8. The film-coated lozenge according to any of claims 1 to 5, for use as a drug.

9. The film-coated lozenge for use according to claim 8, in treating tobacco dependence.
